# EUROPEAN PATENT SPECIFICATION

(11) **EP 4 374 961 B1**
(45) Date of publication and mention of the grant of the patent: **11.02.2026**
(21) Application number: 22845702.4
(22) Date of filing: 06.06.2022
(51) Int. Cl.: B01J 19/12, C02F 1/72, F04D 29/28, A61L 9/00, A61L 9/16, A61L 9/18, A61L 9/20, F24F 8/167

(54) **PHOTOCATALYST UNIT AND METHOD FOR MANUFACTURING PHOTOCATALYST UNIT**
PHOTOKATALYSATOREINHEIT UND VERFAHREN ZUR HERSTELLUNG EINER PHOTOKATALYSATOREINHEIT
UNITÉ DE PHOTOCATALYSEUR ET PROCÉDÉ DE FABRICATION D'UNE UNITÉ DE PHOTOCATALYSEUR

(30) Priority: 21.07.2021 JP 2021120413
(43) Date of publication of application: 29.05.2024
(73) Proprietor: APS Japan Co., Ltd., Osaka-shi, Osaka 541-0059 (JP)
(72) Inventor: WATANABE Teruo, Osaka-shi, Osaka 541-0059 (JP); WATANABE Hidemitsu, Osaka-shi, Osaka 541-0059 (JP); WATANABE Hiroyuki, Osaka-shi, Osaka 541-0059 (JP); WATANABE Takafumi, Osaka-shi, Osaka 541-0059 (JP); YAMAGUCHI Masao, Osaka-shi, Osaka 541-0059 (JP)
(74) Representative: Vossius & Partner Patentanwälte Rechtsanwälte mbB
(86) International application number: PCT/JP2022/022796
(87) International publication number: WO 2023/002765

(56) References cited:
- WO-A1-2017/099231
- JP-A- 2006 305 084
- JP-A- 2010 090 858
- JP-A- 2011 104 490
- JP-A- 2019 214 997
- JP-A- H0 938 189
- US-A1- 2002 172 627
- US-A1- 2017 035 926

## Description

### [Technical Field]

The present invention relates to a photocatalyst unit that cleans fluid such as air with a photocatalyst, and a method for manufacturing the photocatalyst unit.

### [Background Art]

Conventionally, an air cleaner having an air cleaning structure that decomposes viruses and the like using photocatalysts has been proposed (see Patent Literatures (PTLs) 1 and 2, for example). In such a conventional air cleaning structure using photocatalysts, a photocatalyst filter is configured by carrying a photocatalyst such as titanium oxide on one of front and back surfaces of a plate-shaped base material (filter base material) that has a large number of air passage holes penetrating through the thickness of the base material; a photocatalyst unit in which a plurality of ultraviolet (UV) lamps are arranged at intervals at positions facing the surface carrying the photocatalyst in the photocatalyst filter is provided; and air is supplied to the unit from one side of front and back in a thickness direction of the filter, to be circulated through the air passage holes of the filter, a photocatalyst layer, and gaps between the plurality of UV lamps. In a process of the air passing through the photocatalyst layer, the photocatalyst excited by UV light decomposes and removes harmful substances in the air.

In an air cleaning structure using such a conventional photocatalyst unit, the photocatalyst filter and the UV lamps are arranged facing each other in the thickness direction of the filter, thereby increasing the thickness of the unit. This restricts reduction in size and thickness of the structure. In addition, in the structure, air flows between the UV lamps. This restricts the number of UV lamps to be provided due to air resistance. Thus, there is a certain limit to increasing a volume of UV light to be emitted to the photocatalyst filter and to improving efficiency of the emittance. For example, even if the photocatalyst is carried deep inside the air passage hole of the plate-shaped base material, it is difficult to efficiently apply the UV light to the deep inside.

In contrast, an air cleaning structure has been proposed (see PTL 3) in which: a plurality of plate-shaped parts each carrying a photocatalyst on their front and back surfaces are arranged so that the front and back faces of adjacent plate-shaped parts face each other with a gap between them, to configure a photocatalyst filter having the gap serving as an air flow path; a UV light emitting unit that emits UV light toward the gap is provided at a position opposite to at least one of an end face of an inlet for air into the gap and an end face of an outlet for the air from the gap among end faces of each plate-shaped part of the photocatalyst filter, the position being placed at a predetermined distance from the at least one end face; an air supply path or an air discharge path is formed between the UV emitting unit and the end face of each plate-shaped part, the air supply path taking air from a side substantially parallel to the end face and supplying the air to the air flow path formed by the gap between the plate-shaped parts, the air discharge path discharging the air flowing out of the flow path to the side that is substantially parallel to the end face.

In such an air cleaning structure, the UV light emitting unit is arranged at a predetermined distance from the end face of the air inlet or the air outlet among the end faces of the respective plate-shaped parts of the photocatalyst filter, and the air supply path that takes air from a side substantially parallel to the end face or the air discharge path that discharges the air to a side substantially parallel to the end face is formed between the UV light emitting unit and the end face of each plate-shaped part. Thus, the air is not supplied or discharged through the gap between the UV lamps. Therefore, the degree of freedom in designing the UV light emitting unit has been significantly improved, and the UV light is efficiently applied to the photocatalyst in each plate-shaped part, thereby to remarkably improve the air cleaning effect of the photocatalyst.

However, in such an air cleaning structure, air is supplied or discharged through an L-shape form from a side between the UV light emitting unit and the photocatalyst filter. Accordingly, a form (flow path) of air supply/discharge is limited and the unit as a whole will become inevitably larger. Furthermore, in a case where the fluid is desired to flow straight in the thickness direction of the filter instead of the direction forming the L-shape, the above-described configuration cannot be adopted, so that the flow path structure is restricted.

The present applicant has also proposed a fluid cleaning structure using a photocatalyst (PTL 4). The fluid cleaning structure can be formed thinner and smaller, and has a high degree of freedom in designing the flow path structure, as follows. The fluid cleaning structure includes a photocatalyst filter, a light emitting unit, a fluid supply path, a fluid discharge path, and a dust collection filter. The photocatalyst filter is formed of a wave-shaped member with multiple alternating mountain portions and valley portions. In one or both of the top of each mountain portion and the bottom of each valley portion, a fluid passage hole for allowing the fluid to pass through is formed. The photocatalyst filter carries a photocatalyst on its front and back surfaces. The light emitting unit is provided on one or both of one end side and the other end side of the photocatalyst filter in a direction along which the mountain portions and the valley portions extend. The light emitting unit emits UV light or visible light in an inward direction along which the mountain portions and the valley portions extend. The fluid supply path is provided in one surface side of the front and back surfaces of the photocatalyst filter, and supplies fluid toward the one surface. The fluid discharge path is provided in the other surface side of the front and back surfaces of the photocatalyst filter, and discharges fluid that is supplied from the fluid supply path, passes through the fluid passage hole, and exits from the other surface. The dust collection filter is provided inside one or both of the fluid supply path and the fluid discharge path, and is provided facing the one surface or the other surface.

According to such a fluid cleaning structure, the light emitting unit is not provided at a position facing the filter surface as in the conventional case, but is provided on one or both of one end side and the other end side in the direction along which the mountain portions and valley portions of the photocatalyst filter extend. The light emitting unit is configured to emit the UV light or visible light from that position in an inward direction along which the mountain portions and valley portions extend. Accordingly, it is possible to allow the fluid to flow straight in the thickness direction of the filter without being obstructed by the light emitting unit, thereby increasing the degree of freedom in designing the flow path, and making the unit including the filter and light emitting unit significantly thinner and more compact.

As described above, the light is emitted inward from the end of the photocatalyst filter along the direction in which the mountain portions and valley portions extend. Thus, in order to obtain a filter effect, an area of the photocatalyst filter may be increased and the mountain portions and valley portions may become longer, making it difficult for the light to sufficiently reach deep inside. In particular, the light becomes insufficient in the mountain portions and valley portions located away from the position of the light emitting unit. Accordingly, it is necessary to increase the volume of light or increase the number of the light emitting units, which poses problems of increased cost and heat generation.

PTL 5 discloses a fan with an air purification function using a photocatalytic reaction.

### [Citation List]

### [Patent Literature]

[PTL 1] Japanese Utility Model Registration No. 3150894
[PTL 2] Japanese Unexamined Patent Application Publication (Translation of PCT Application) No. 2011-114894
[PTL 3] Japanese Unexamined Patent Application Publication No. 2017-148484
[PTL 4] Japanese Unexamined Patent Application Publication No. 2021-7676
[PTL 5] JP H09 38189 A
[PTL 6] Japanese Unexamined Patent Application Publication No. 2020-090858A
[ PTL 7] WO 2017/099231 A1
[PTL 8] Japanese Unexamined Patent Application Publication No. 2019-214997 A

### [Summary of Invention]

### [Technical Problem]

In view of the above-described situation, a purpose of the present invention is to provide a photocatalyst unit that (i) can improve a fluid cleaning effect of the photocatalyst by efficiently emitting UV light to the photocatalyst, (ii) can be formed thin and compact as a photocatalyst unit including a photocatalyst filter and a light emitting unit, (iii) has a high degree of freedom in designing a flow path structure, and (iv) can obtain a sufficient cleaning effect as a whole without increasing a volume of light or the number of light sources.

### [Solution to Problem]

In view of the current situation, the present inventor has intensively studied and found the following. Unlike conventional photocatalyst filter having a fixed corrugated plate with mountain portions and valley portions, an impeller is provided in which a plurality of blade plates each carrying a photocatalyst rotate under pressure from fluid or are driven by a motor and the like, and light is emitted from an outer peripheral side to the rotating blade plates of the impeller, thereby emitting the light sequentially and uniformly on a surface of each blade plate. Therefore, light can be supplied uniformly and efficiently to the photocatalyst carrier with a small number of light emitting units. It is also possible to obtain a sufficient cleaning effect as a whole without increasing a volume of the light or increasing the number of light sources. Furthermore, the inventor has devised a rotating body structure with excellent rigidity that would not cause problems even when rotated at high speeds, and has completed the present invention.

Specifically, the present invention is defined by the claims. This disclosure includes the following aspects.
(1) A photocatalyst unit including: a photocatalyst filter including a fluid passage through which fluid passes, and a photocatalyst carrier, in the fluid passage, having a surface on which a photocatalyst is carried, the surface being in contact with the fluid, and a light emitting unit that is provided inside the photocatalyst filter and emits ultraviolet (UV) light or visible light to the surface of the photocatalyst carrier, the surface carrying the photocatalyst, in which the photocatalyst carrier includes a plurality of blade plates that are made of metal, each of the plurality of blade plates having a surface on which the photocatalyst is carried, the plurality of blade plates constituting one of a blower fan like impeller and a cross flow fan like impeller, which rotate about a common axis in response to pressure from the fluid passing through the fluid passage, or one of an impeller serving as a blower fan and an impeller serving as a cross flow fan, which rotate about a common axis by a motor drive, the impeller includes a pair of connecting plates that connect to each of the blade plates at both ends thereof in the axial direction, each of the blade plates and the connecting plates are fixed in a manner that a part of each of the both ends of the blade plate penetrates and protrudes through a through groove formed in each of the connecting plates, and the part protruding is bent in a direction intersecting the axial direction and parallel to the surface of the blade plate so as to be crimped and fixed to an opening periphery of the through groove of the connecting plate, and the light emitting unit is provided to emit the light to the blade plates of the impeller from a predetermined position in the impeller on an outer peripheral side from the blade plates, the blade plates being rotating.
(2) The photocatalyst unit according to (1) in which the photocatalyst is also carried on a surface of each of the pair of connecting plates that constitute the impeller.
(3) The photocatalyst unit according to (1) or (2), in which the photocatalyst filter includes a rectifier plate that is made of metal and extends in a circumferential direction on the outer periphery side of the impeller to form the fluid passage with the impeller, and a pair of upper and lower cover plates respectively fixed to both ends of the rectifier plate in the axial direction to have the rectifier plate interposed therebetween, and the rectifier plate and each of the cover plates are fixed in a manner that a part of each of the both ends of the rectifier plate penetrates and protrudes through a through groove formed in each of the cover plates, and the part protruding is bent in a direction intersecting the axial direction and parallel to a surface of the rectifier plate so as to be crimped and fixed to an opening periphery of the through groove of the cover plate.
(4) The photocatalyst unit according to (3), in which the light emitting unit that is provided at a position outside the rectifier plate, and emits the light toward an axis of the impeller provided inside the rectifier plate through a projection through hole that is provided in the rectifier plate for projecting the light.
(5) The photocatalyst unit according to any one of (1) to (4), in which the rectifier plate is placed on a straight line between a center point of an opening surface of a fluid outlet provided in an outer circumferential direction of the blade plates and a center point of an inner opening surface of the projection through hole formed in the rectifier plate, so as to prevent the light emitted inward through the projection through hole from directly exiting from the fluid outlet.
(6) A method for manufacturing a photocatalyst unit that includes: a photocatalyst filter including a fluid passage through which fluid passes, and a photocatalyst carrier, in the fluid passage, having a surface on which a photocatalyst is carried, the surface being in contact with the fluid, and a light emitting unit that is provided inside the photocatalyst filter and emits UV light or visible light to the surface of the photocatalyst carrier, the surface carrying the photocatalyst, the method including: allowing the photocatalyst carrier to include a plurality of blade plates that are made of metal, each of the plurality of blade plates having a surface on which the photocatalyst is carried, the plurality of the blade plates constituting one of a blower fan like impeller and a cross flow fan like impeller, which rotate about a common axis in response to pressure from the fluid passing through the fluid passage, or one of an impeller serving as a blower fan and an impeller serving as a cross flow fan, which rotate about a common axis by a motor drive; allowing the impeller to include a pair of connecting plates that connect to each of the blade plates at both ends in the axial direction; fixing the blade plate and the connecting plates by allowing a part of each of the both ends of the blade plate to penetrate and protrude from a through groove formed in each of the connecting plates, and bending the part in a direction intersecting the axial direction and parallel to the surface of the blade plate to fix the part to an opening periphery of the through groove of the connecting plate by caulking; and providing the light emitting unit to emit the light to the blade plates of the impeller from a predetermined position in the impeller on an outer peripheral side from the blade plates, the blade plates being rotating.
(7) The method for manufacturing a photocatalyst unit according to (6), including: roughening an entire surface of the impeller including each blade plate and the pair of connecting plates by roughening processing including etching processing, and causing the photocatalyst to be carried on the surface roughened.
(8) The method for manufacturing a photocatalyst unit according to (7), including: performing another caulking on a portion fixed by the caulking previously performed after the roughing.
(9) The method for manufacturing a photocatalyst unit according to any one of (6) to (8), including: allowing the photocatalyst filter to include a rectifier plate that is made of metal and extends in a circumferential direction on the outer peripheral side of the impeller to form the fluid passage with the impeller, and a pair of upper and lower cover plates respectively fixed to both ends of the rectifier plate in the axial direction to have the rectifier plate interposed therebetween, and fixing the rectifier plate and the cover plates by allowing a part of each of the both ends of the rectifier plate to penetrate and protrude from a through groove formed in each of the cover plates, and bending and deforming the part in a direction intersecting the axial direction and parallel to the surface of the rectifier plate to fix the part to an opening periphery of the through groove of the cover plate by caulking.

### [Advantageous Effects of Invention]

According to the present invention configured as above, the plurality of blade plates carrying photocatalysts are irradiated with the UV light or visible light from the light emitting unit provided on the outer periphery side of the blade plates. With this configuration, harmful substances, bad odors, and the like in fluid can be efficiently decomposed and removed by the photocatalyst carried on a surface of the filter. Furthermore, the light emitting unit is provided at a position in the outer peripheral side of the blade plates, so that the fluid can flow without being obstructed by the light emitting unit. Therefore, the degree of freedom in designing the flow path is improved, and the unit including the filter and the light emitting unit can be made significantly thinner and more compact.

According to the present invention, the light from the light emitting unit can be applied sequentially and uniformly to the surface of the rotating blade plates, thereby uniformly and efficiently supplying the light to the photocatalyst carrier with a small number of the light emitting units. Accordingly, a sufficient cleaning effect as a whole can be obtained without increasing a volume of the light or the number of the light emitting units. Therefore, it is possible to avoid increase in cost, and prevent increase in a quantity of heat generated by the light emitting unit so as to solve a problem of the heat. It is efficient for the light emitting unit to emit light from a predetermined position in the outer peripheral side of the blade plates toward the shaft of the impeller in order to apply the light to both surfaces of each blade plate.

In particular, the impeller has a pair of metal connecting plates that connect to each blade plate at its both ends in the axial direction, and each blade plate and the connecting plates are connected in a manner that a part of each end of the blade plate penetrates and protrudes through a through groove formed in each connecting plate, and the protruding portion is bent in a direction intersecting the axial direction and parallel to the surface of the blade plate, so as to be fixed in a crimped state to an opening periphery of the through groove of the connecting plate. Accordingly, in the fixed structure, the bent and crimped part is bent in a direction parallel to the plate surface. Therefore, it is difficult to loosen due to stress release and the like, excellent connection strength can be maintained, and a stable posture can be maintained without the blade plate being fallen down even under wind pressure. This allows the impeller to have excellent rotational balance as designed, and more excellent cleaning effect can be obtained by rotating at a higher speed.

Such a fixing structure of each blade plate by crimping is efficiently achieved by caulking. As described later, the fixed structure can maintain its strength even if the part bent in a direction parallel to the plate surface undergoes the caulking twice. After first caulking (temporary caulking), etching or the like may be performed as a pretreatment to carry the photocatalyst on the surfaces of the blade plates. In such a situation, even if the fixed part loosens, the strength can be maintained by second caulking.

Here, in a case where the photocatalyst is also carried on each surface of the pair of connecting plates constituting the impeller, the cleaning effect can be enhanced. In addition, it is possible to efficiently perform the pretreatment including etching and the carrying of the photocatalyst on the entire impeller including the connecting plates at once, by performing caulking twice as described above.

Furthermore, the photocatalyst filter may include the metal rectifier plate and the pair of upper and lower cover plates. The rectifier plate is provided to extend in the circumferential direction on the outer peripheral side of the impeller, and forms a fluid passage with the impeller. The pair of upper and lower cover plates are fixed to both ends of the rectifier plate in the axial direction and have the rectifier plates interposed therebetween. The rectifier plates and the cover plates are fixed in a state where a part of each end of the rectifier plate penetrates and protrudes through a through groove formed in each cover plate, and the protruding portion is bent in a direction intersecting the axial direction and parallel to the surface of the rectifier plate, to be fixed in a crimped state to the opening periphery of the through groove of the cover plate. In such a photocatalyst filter, the rectifier plate that forms the fluid passage also has the same structure as the structure used to fix the blade plates to the connecting plates. Therefore, looseness is less likely to occur, and excellent connection strength to withstand wind pressure can be maintained so as to maintain a stable posture even under the wind pressure. Therefore, dimensional accuracy can be maintained, and a gap between the impeller and the rectifier plate can be kept to minimum, thereby allowing a product to be thinner and more compact.

The light emitting unit may be provided at an outside position of the rectifier plate, and emits the light toward the axial center of the impeller placed in an inner side, through a through hole for light projection, which is formed in the rectifier plate. In such a configuration, the light emitting unit does not interfere with flow of the fluid inside the rectifier plates, thereby further reducing the pressure loss. In addition, deterioration by adhesion of dust due to being placed in a fluid flow can be avoided. Such a light emitting unit can be configured independently from the housing structure including the rectifier plate and the cover plates. Therefore, processing of wiring relating to the light emitting unit is easily performed in comparison with a case where the light emitting unit is arranged inside the rectifier plate, thereby simplifying both manufacturing operation and assembly operation.

The rectifier plate may be placed between a center point of an opening surface of a fluid outlet provided at a position in the outer circumferential direction of the blade plate and a center point of an inner opening surface of the through hole for light projection formed in the rectifier plate, on a straight line connecting the two center points, so that the light emitted inward from the through hole does not directly exit from the fluid outlet. In such a configuration, it is possible to more reliably prevent UV light harmful to the human bodies from leaking from the fluid outlet.

The photocatalyst carrier may be a blower-fan like impeller or an impeller serving as a blower fan. The fluid passage may include a fluid introduction part that introduces the fluid from one side in a direction along the axis or in a direction perpendicular to the axis, and a fluid lead-out part that leads the fluid toward the other side in the direction along the axis or in a direction perpendicular to the axis. In such a structure, the fluid passing through the fluid passage is brought into contact with the photocatalyst carried on a surface of each blade plate while light is emitted from the light emitting unit toward the rotating blade plates, thereby efficiently decomposing and removing harmful substances and bad odors in the fluid by a catalytic action of the photocatalyst. In addition, a flowing direction of the fluid passing through the fluid passage is changed to a direction perpendicular to the axis, as described, and the fluid temporarily stagnates between the rotating blade plates, thereby increasing opportunities of contact between the fluid and the surface of each blade plate, which carries the photocatalyst. Accordingly, the catalyst action described above can be enhanced. Furthermore, the fluid lead-out part is provided at a position in the direction perpendicular to the fluid introduction part as described above, thereby increasing design variation.

The photocatalyst carrier may be a cross flow fan-like impeller or an impeller serving as a cross flow fan. The fluid passage may have a fluid introduction part that introduces the fluid from a direction perpendicular to the axis, and a fluid lead-out part that leads the fluid out from a position different from the fluid introduction part in the direction perpendicular to the axis. In such a configuration, harmful substances and bad odors in the fluid can be efficiently decomposed and removed by the catalytic action of the photocatalyst, and a period during which the fluid temporarily remains between the rotating blade plates can be longer than that of the blower fan-like impeller or the impeller serving as a blower fan. Accordingly, the opportunities of contact between the fluid and the surface of each blade plate, which carries the photocatalyst are increased, thereby enhancing the catalytic action. In addition, a dimension of the blade plate in the axial direction can be freely set, so that the fluid passage can be made larger in the axial direction, and can be installed efficiently even in relatively large channels. Thus, it is easy to keep the flow resistance lower.

Each blade plate and the connecting plate may be fixed in a method in which a part of the end of each blade plate penetrates and protrudes through a through groove formed in each connecting plate, and the protruding portion is bent and deformed by caulking in a direction intersecting the axial direction and parallel to the surface of the blade plate, to be fixed to the opening periphery of the through groove of the connecting plate. In such a manufacturing method, in the caulking, the blade plates are bent in parallel to the plate surface and crimped, thereby maintaining its strength even with undergoing caulking twice. After the first caulking (temporary caulking), etching is performed as pretreatment to carry the photocatalyst on the surfaces of the blade plates. Even if the fixed part loosens, the strength can be maintained by performing the second caulking.

Generally, the etching processing causes a board thickness to be reduced and a caulked parts are loosened. Accordingly, when performing pretreatment such as etching, a method of crimping, such as caulking, to be performed between members is incompatible and is not adopted. However, in the present invention, a fixed structure that can be caulked twice as described above is adopted. Accordingly, unlike normal methods, it is possible to adopt the caulking method even when performing the pretreatment described above. The fixation by the caulking is enabled, thereby increasing the number of the blade plates that carry the photocatalyst, and maximizing a surface area of the photocatalyst that is exposed to UV light. This makes it easier to enhance the cleaning effect. Even if a device is minimized, satisfactory decomposition performance can be secured.

The entire surface of the impeller including the blade plates and the pair of connecting plates may be roughened by surface roughening treatment including the etching processing. In the manufacturing method in which the photocatalyst is carried on the roughened surface, the photocatalyst can be efficiently carried.

When the caulked parts are again caulked after the surface has been roughened, the strength can be maintained. According to the present invention in which recaulking can be performed, processing of carrying the photocatalyst can be efficiently performed.

### [Brief Description of Drawings]

[FIG. 1]

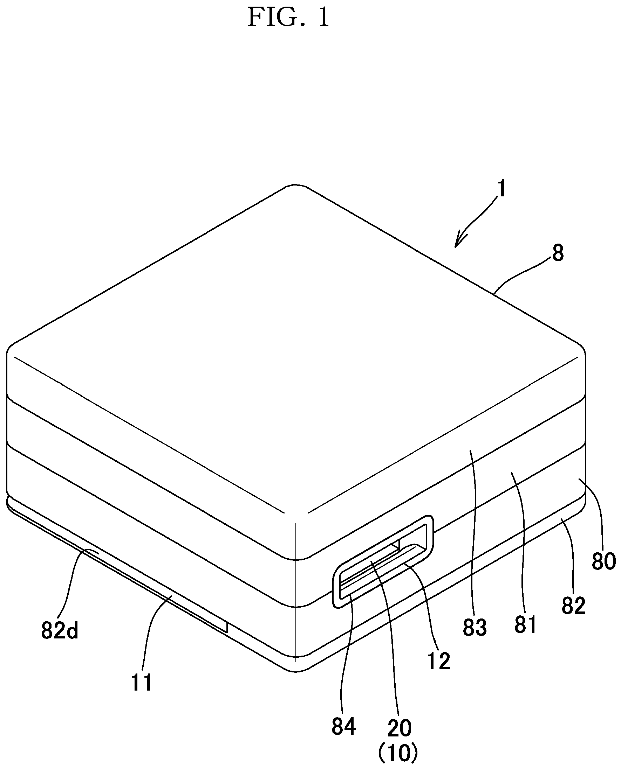

   FIG. 1 is a perspective view showing a photocatalyst unit according to a typical embodiment of the present invention.
[FIG. 2]

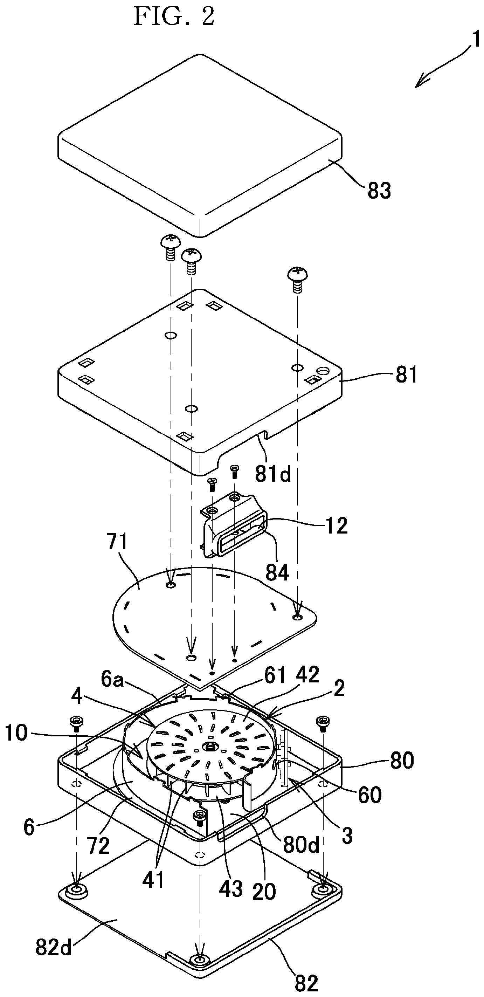

   FIG. 2 is an exploded perspective view of the photocatalyst unit.
[FIG. 3]

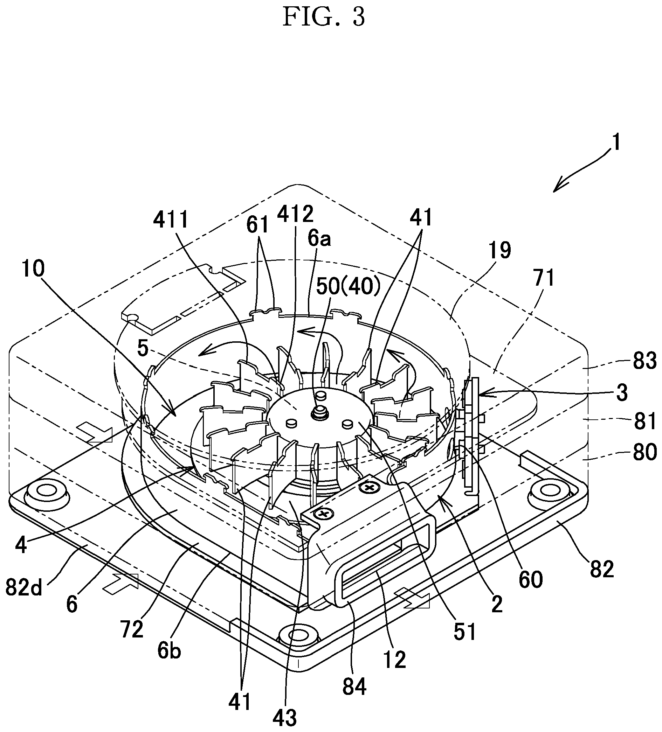

   FIG. 3 is a partially omitted perspective view of the photocatalyst unit.
[FIG. 4]

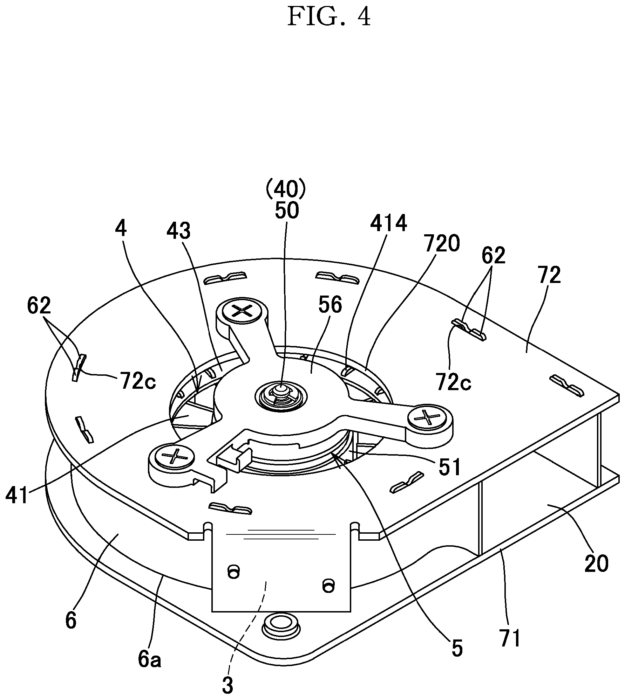

   FIG. 4 is a perspective view showing a photocatalyst filter inside the photocatalyst unit.
[FIG. 5A]

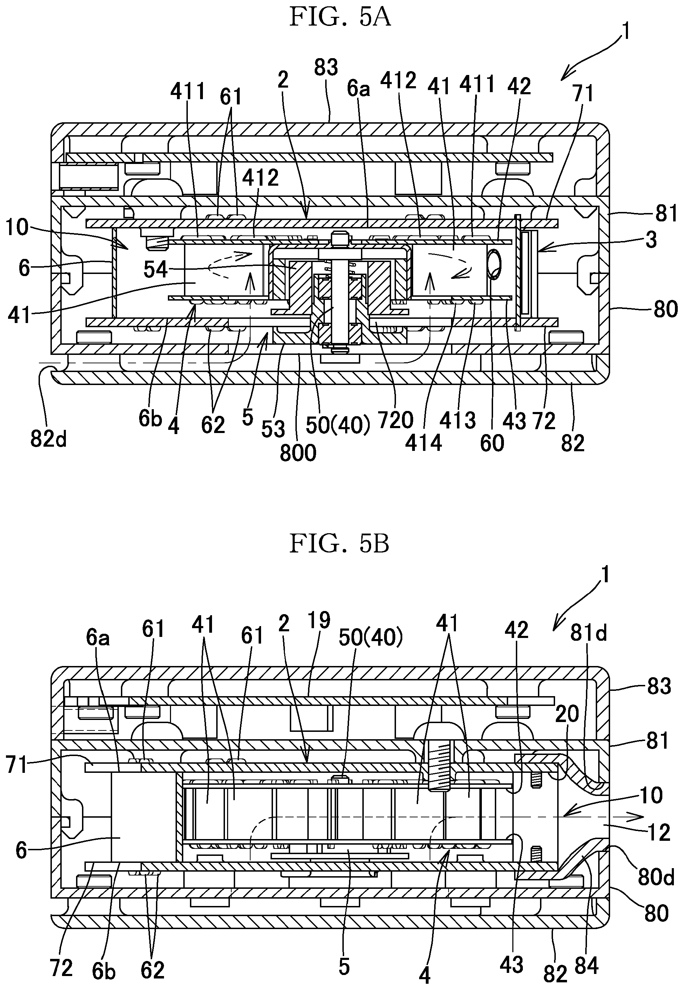

   FIG. 5A is a longitudinal sectional view of the photocatalyst unit.
[FIG. 5B]
   FIG. 5B is a longitudinal sectional view of the photocatalyst unit.
[FIG. 5C]

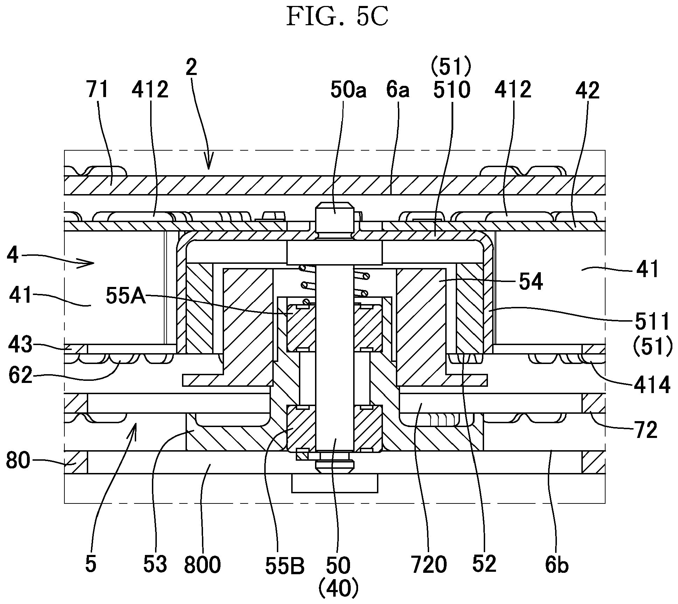

   FIG. 5C is a longitudinal sectional view of a main part of the photocatalyst unit.
[FIG. 6]

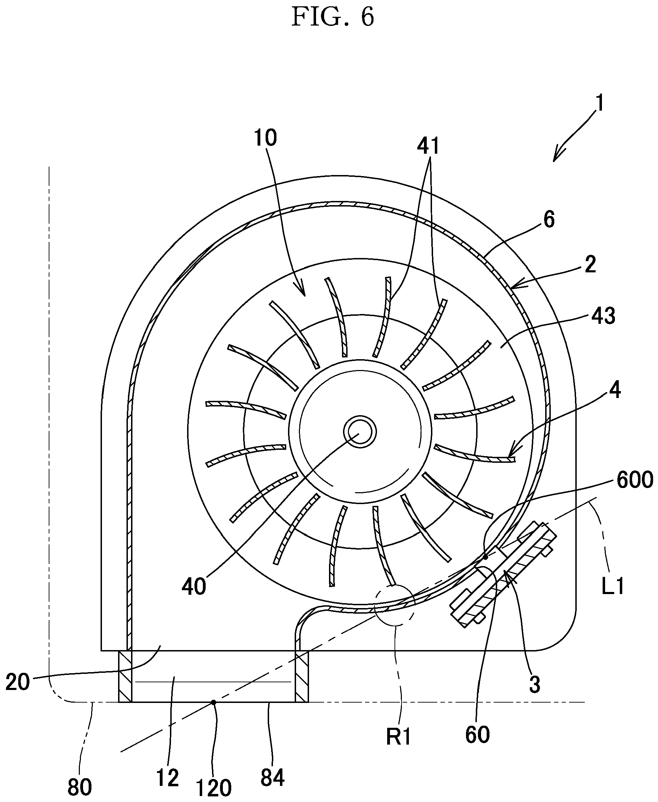

   FIG. 6 is a cross sectional view showing an internal structure of the photocatalyst unit.
[FIG. 7]

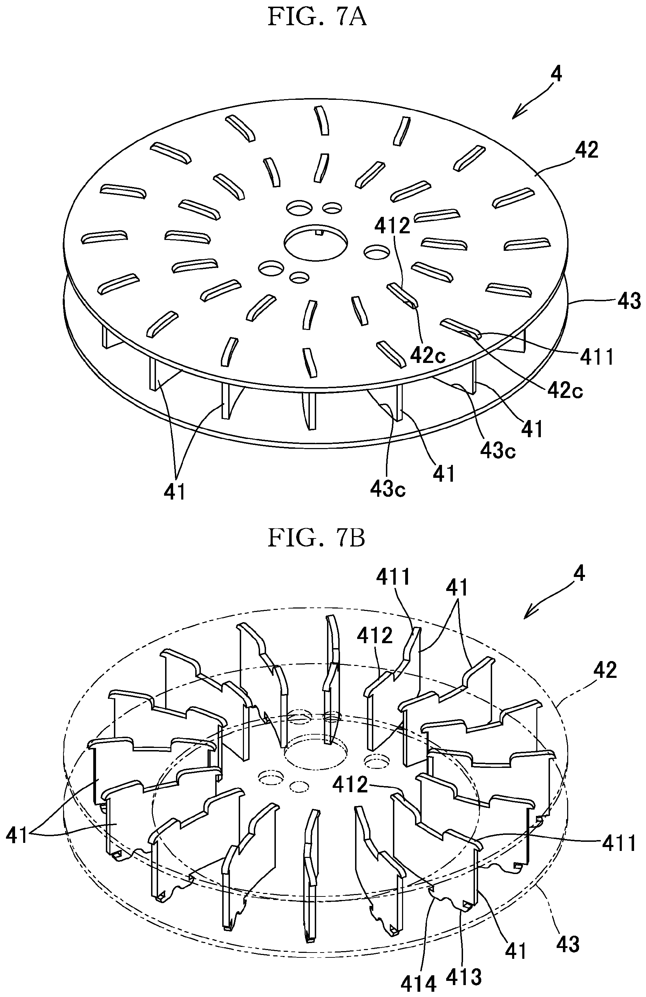

   FIG. 7A is a perspective view showing an impeller (photocatalyst carrier) inside the photocatalyst filter, and FIG. 7B is a perspective view showing an arrangement of blade plates of the impeller.
[FIG. 8]

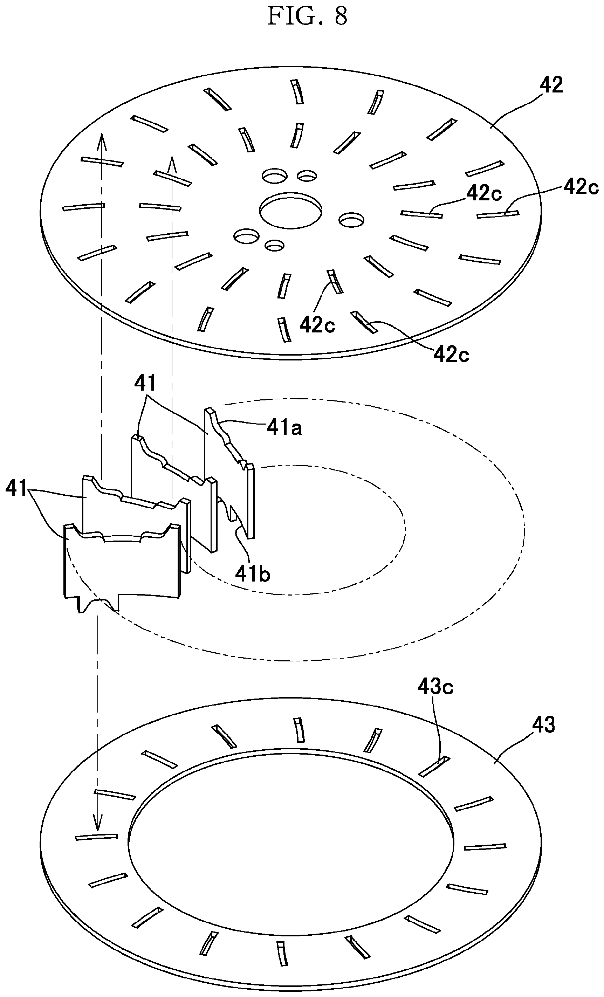

   FIG. 8 is an explanatory diagram showing a manufacturing procedure of the impeller.
[FIG. 9A]

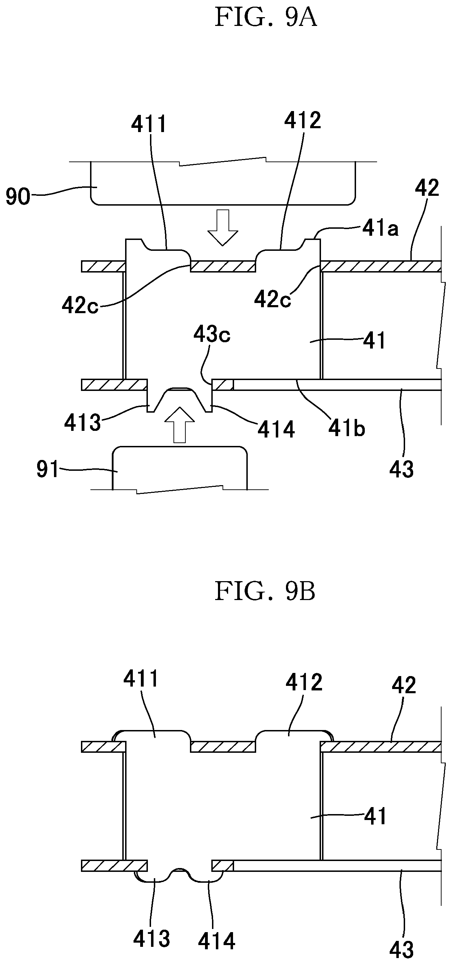

   FIG. 9A is an explanatory diagram of the manufacturing procedure.
[FIG. 9B]
   FIG. 9B is an explanatory diagram of the manufacturing procedure.
[FIG. 9C]

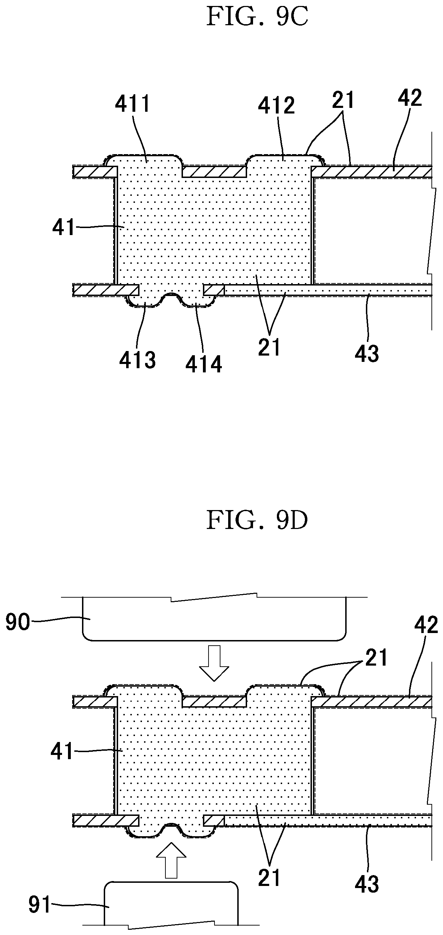

   FIG. 9C is an explanatory diagram of the manufacturing procedure.
[FIG. 9D]
   FIG. 9D is an explanatory diagram of the manufacturing procedure.
[FIG. 10A]

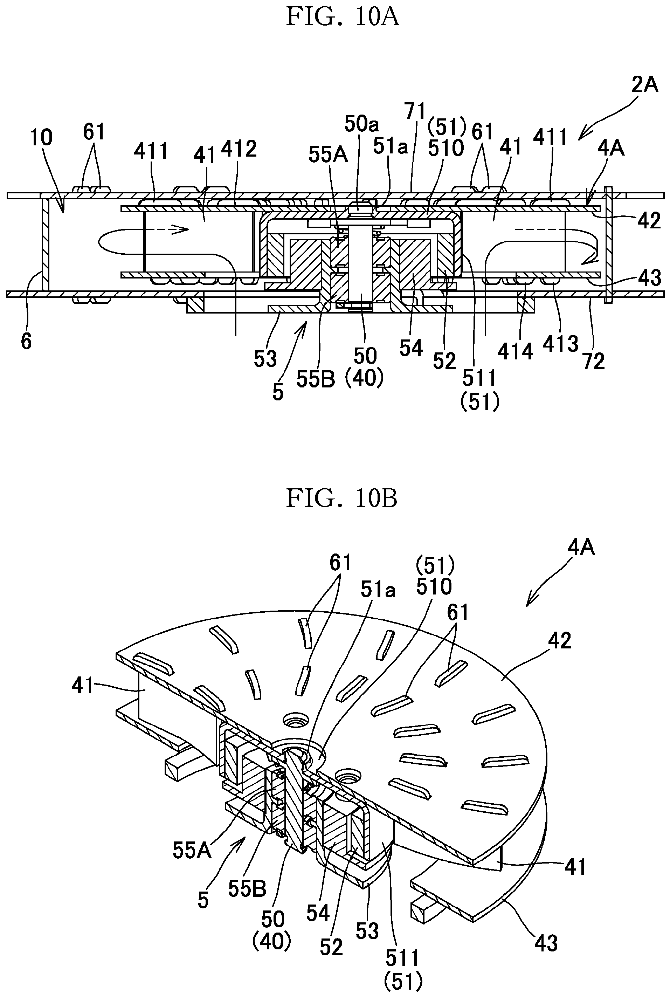

   FIG. 10A is a partially cutaway perspective view showing a modified example of the photocatalyst unit according to the present invention.
[FIG. 10B]
   FIG. 10B is a longitudinal sectional view of the modified example.
[FIG. 11]

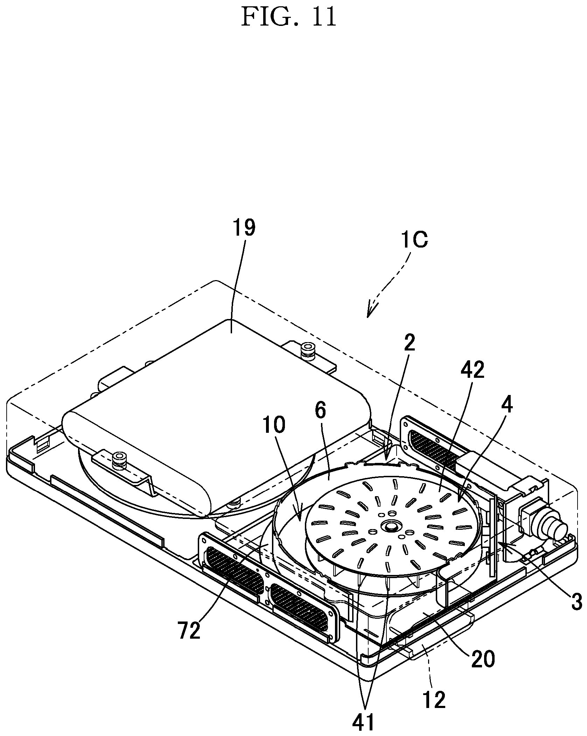

   FIG. 11 is a perspective view showing an internal structure of another modified example of the photocatalyst unit according to the present invention.
[FIG. 12]

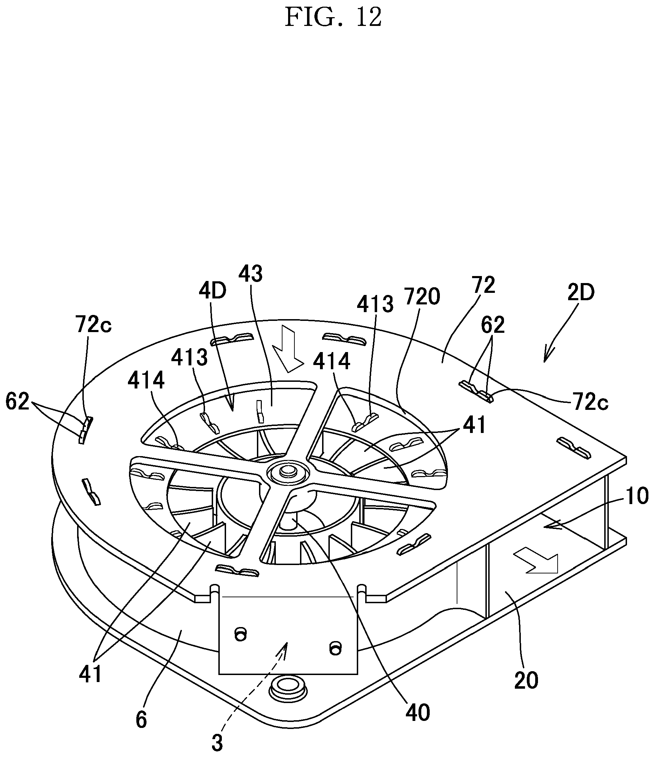

   FIG. 12 is a perspective view showing a photocatalyst filter according to the other modified example of the photocatalyst unit according to the present invention.
[FIG. 13]

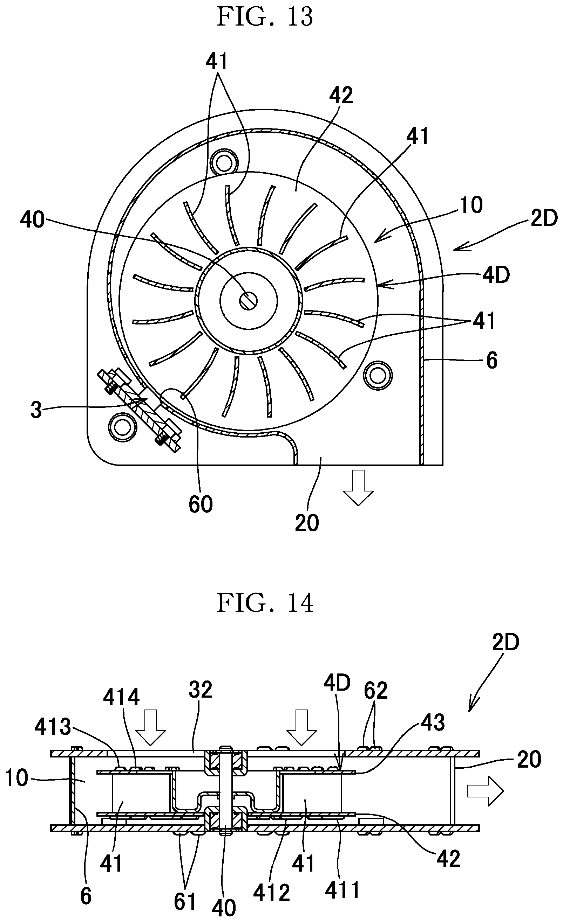

   FIG. 13 is a cross sectional view of the photocatalyst filter according to the other modified example.
[FIG. 14]
   FIG. 14 is a longitudinal sectional view of the photocatalyst filter according to the other modified example.
[FIG. 15]

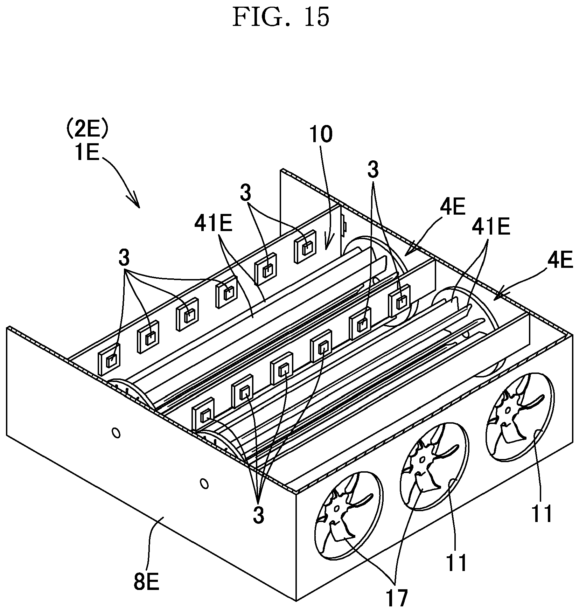

   FIG. 15 is a perspective view showing an internal structure of still another modified example of the photocatalyst unit according to the present invention.
[FIG. 16]

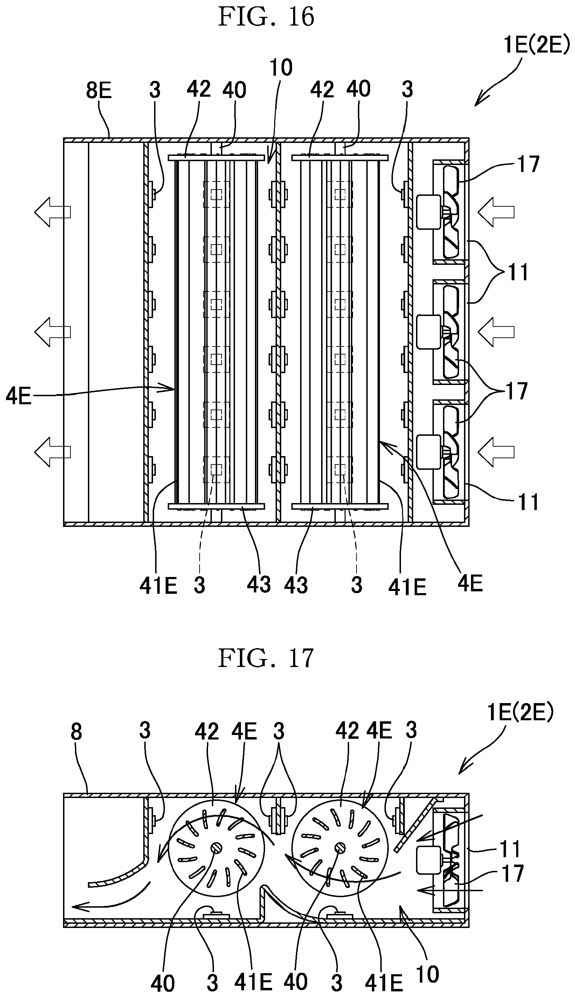

   FIG. 16 is a cross sectional view showing the internal structure of the still other modified example.
[FIG. 17]
   FIG. 17 is a longitudinal sectional view showing the internal structure of the still other modified example.
[FIG. 18]

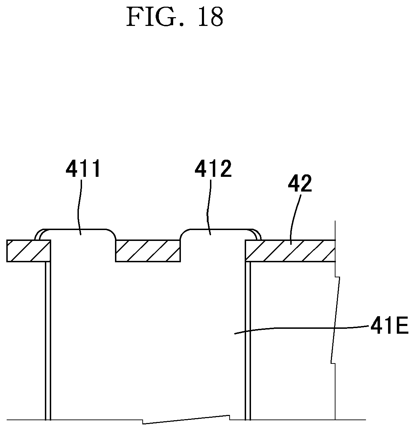

   FIG. 18 is an explanatory diagram of a main part of the still other modified example.
[FIG. 19A]

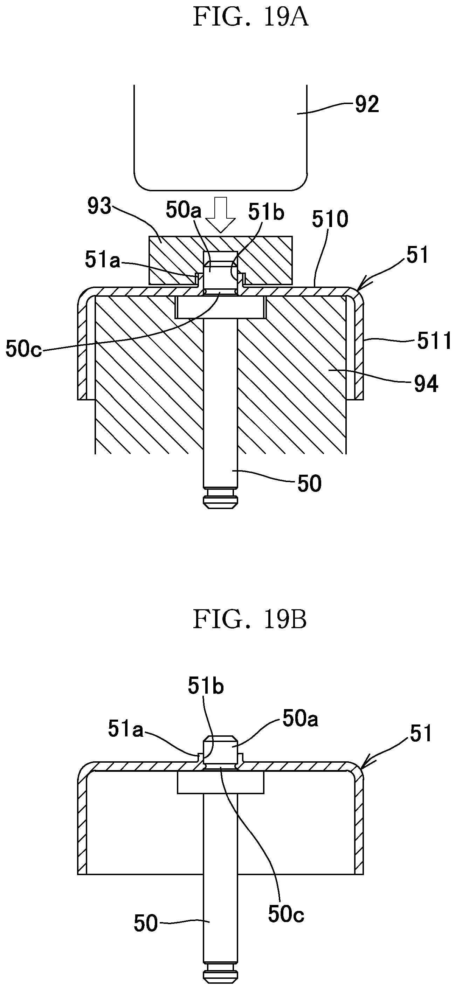

   FIG. 19A is an explanatory diagram showing caulking on a motor housing and a shaft.
[FIG. 19B]
   FIG. 19B is an explanatory diagram showing the caulking on the motor housing and the shaft.
[FIG. 20]

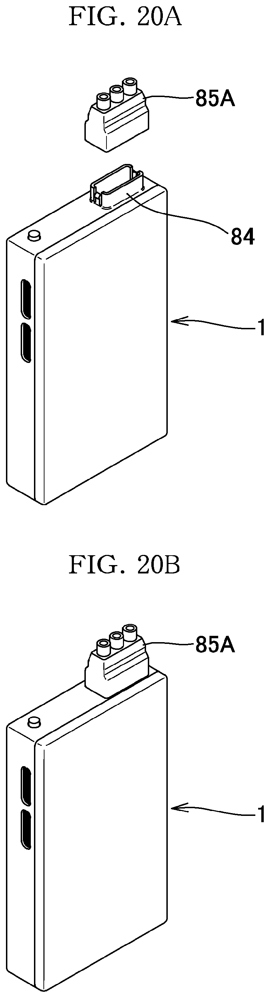

   FIG. 20 is an explanatory diagram showing a usage pattern.
[FIG. 21]

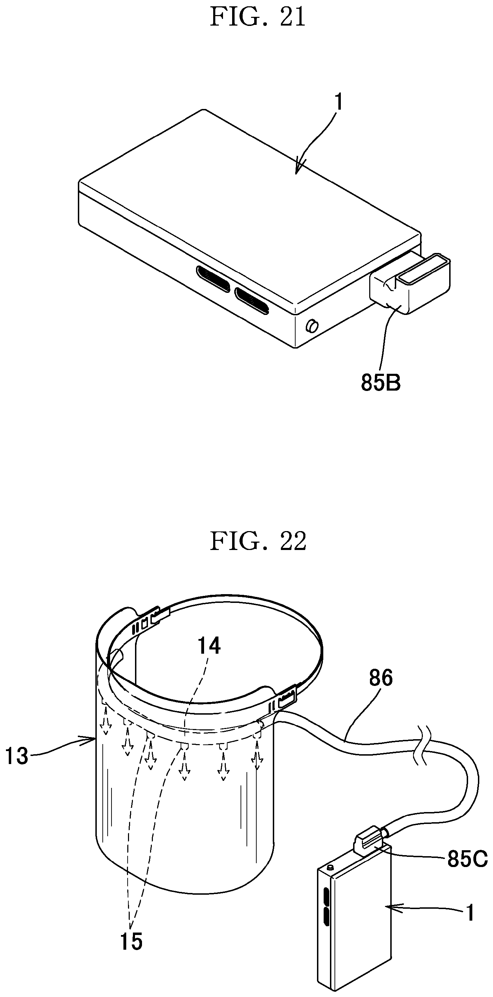

   FIG. 21 is an explanatory diagram showing another usage pattern.
[FIG. 22]
   FIG. 22 is an explanatory diagram showing still another usage pattern.

### [Description of Embodiments]

Next, embodiments of a photocatalyst unit according to the present invention will be described with reference to the accompanying drawings.

A photocatalyst unit 1 according to a typical embodiment of the present invention includes, as shown in FIGS. 1 to 5C, a photocatalyst filter 2 and a light emitting unit 3, inside a housing 8 having a fluid inlet 11 and a fluid outlet 12. The photocatalyst filter 2 includes a photocatalyst carrier (impeller 4) with a photocatalyst carried on its surface along which the fluid passes. The light emitting unit 3 emits UV light or visible light to the surface of the photocatalyst carrier on which the photocatalyst is carried. The UV light or visible light is emitted from the light emitting unit 3 to the surface of the photocatalyst carrier (impeller 4) on which the photocatalyst is carried, so that harmful substances and bad odors in the fluid can be efficiently decomposed and removed by the photocatalyst.

The photocatalyst filter 2 includes a fluid passage 10 through which the fluid passes, and within the fluid passage 10, the photocatalyst carrier (impeller 4) on which the photocatalyst is carried on its surface that comes into contact with the fluid. Furthermore, the light emitting unit 3 is provided inside the photocatalyst filter 2 and emits the UV light or the visible light to a plurality of blade plates 41 on which the photocatalyst is carried, from a position in an outer peripheral side of the blade plates 41.

The light from the light emitting unit 3 is applied to the surfaces of the rotating blade plates 41 sequentially and uniformly. Accordingly, it is possible to uniformly and efficiently supply light to the photocatalyst carrier with a small number of the light emitting unit 3, and to obtain a sufficient cleaning effect as a whole without increasing a volume of the light or increasing the number of light emitting units. Therefore, increase in cost can be avoided, a quantity of heat generated by the light emitting unit can be prevented from increasing, so that a problem relating to the heat can be solved.

Target fluids include air, water, various gases and liquids, and other fluids. For a photocatalyst and UV or visible light to be applied to the photocatalyst, various types of them having photocatalytic action can be used. A wide range of photocatalysts can be used, such as UV-light excited photocatalysts including titanium oxide, and visible-light excited photocatalysts containing tungsten trioxide as a main component, and the like. A method of carrying the photocatalyst (a method of forming a photocatalyst layer) is not particularly limited, but a slurry immersion impregnation method that does not incur many costs is preferred. Other methods such as an immersion impregnation method, a vacuum impregnation method, and a sol-gel method can also be used.

For the light emitting unit 3, a light emitting diode (LED) board having an LED element is used as a light source that emits light using electric power supplied from a power supply or the like (not shown), for example, but is not limited thereto. In conventional photocatalyst filters, it is necessary to provide, depending on a size of the surface of the photocatalyst carrier, a sufficient number of light emitting units that emit light to the surface of the photocatalyst carrier so that the entire surface of the photocatalyst carrier can be irradiated at the same time. Here, in the present invention, as described above, the photocatalyst carrier including the impeller 4 rotates. When the blade plates 41 and an axis 40 approach the installation portion of the light emitting unit 3, the surfaces of the blade plates 41 are sequentially irradiated with the light from the light emitting unit 3. Therefore, the light can be applied uniformly to the entire surface of every blade plate 41, by providing only one or two light emitting units 3.

The photocatalyst carrier (impeller 4) according to the present embodiment has a plurality of metal blade plates 41 each having a surface on which the photocatalyst is carried, and these plurality of blade plates 41 includes the impeller 4 serving as a blower fan, which is driven by a motor 5 so as to rotate around the common axis 40.

More specifically, as shown in FIGS. 7A and 7B, the impeller 4 includes a pair of connecting plates 42 and 43 that connect to each blade plate 41 at its both ends in the axial direction. Each blade plate 41 and the respective connecting plates 42 and 43 are connected in a manner that a part of each end portion of each blade plate 41 (an upper end 41a and a lower end 41b in the vertical direction in the drawing) penetrates and protrudes through each of through grooves 42c and 43c respectively formed in the connecting plates 42 and 43, and protruding portions (protrusions 411 and 412 in the upper end 41a, protrusions 413 and 414 in the lower end 41b) are bent in a direction intersecting the axial direction and parallel to the surface of the blade plate, to be fixed in a crimped state to an opening periphery of each of the through groove 42c and 43c of the respective connecting plates 42 and 43.

In such a fixing structure of the blade plates 41 and the connecting plates 42 and 43, the bent and crimped portions (protrusions 411 to 414) are bent in a direction parallel to the plate surface. Accordingly, the structure is resistant to loosening due to stress release, and the like, and maintains excellent connection strength. Therefore, even if the rotation speed is increased and wind pressure is increased, the blade plates will not fall down and maintain a stable posture so as to maintain excellent rotational balance as designed. Therefore, the impeller 4 can be rotated at a higher speed, and an excellent cleaning effect can be obtained.

The fixation of each blade plate 41 and the connecting plates 42 and 43 can be achieved by performing a caulking method in which a part of each of the end portions 41a and 41b of the blade plates 41 is made to penetrate and protrude through each of the through grooves 42c and 43c respectively provided in the connecting plates, and a support tool (not shown) and pressure punches 90 and 91 are used, to bend and deform the protrusions 411, 412, 413 and 414 in a direction that intersects the axial direction and parallel to the surfaces of the blade plates to allow the protrusions to be fixed to the respective opening peripheries of the through grooves 42c and 43c, as shown in FIGS. 8, 9A, and 9B.

Although it is possible to carry the photocatalyst on the blade plates 41 at a stage before each component is fixed to the connecting plates 42 and 43, it is preferable to carry the photocatalyst on the blade plates 41 after the fixation, to prevent the photocatalyst carried at a state of the components from falling off during the above-mentioned fixation, which may cause quality deterioration. When the photocatalyst is carried after the fixation, the photocatalyst can be carried not only on the blade plates 41 but also on the entire surface of the impeller 4 together with the connecting plates 42 and 43. Here, when the photocatalyst is carried, it is preferable to roughen the surface by roughening treatment including etching treatment, and then to carry the photocatalyst on the roughened surface.

During the surface roughening processing, the fixed portions between the blade plates 41 and the connecting plates 42 and 43 may become thinner and loosen due to etching. However, the fixing structure of the blade plates 41 and the connecting plates 42, 43 according to the present invention allows the portions bent and deformed parallel to the plate surface to maintain their strength even if the portions are caulked twice. Accordingly, pretreatment for carrying a photocatalyst is performed on an entire surface of the impeller 4 including the blade plates 41 and the connecting plates 42 and 43 and then the photocatalyst 21 is actually carried on the entire surface, as shown in FIG. 9C, after the first caulking (temporary caulking) as shown in FIGS. 8, 9A, and 9B. Finally, second caulking is performed as shown in FIG. 9D, and thus the fixing structure that maintains strength can be achieved.

As a material for the blade plates 41, various metal materials such as aluminum, stainless steel, titanium, and the like can be used. The axis 40 and the connecting plates 42 and 43 are also preferably made of metal of these materials, but are not limited thereto.

A motor 5 that rotationally drives the impeller 4 is attached to the impeller 4 at the center of an inner surface of the connecting plate 42. Specifically, as shown in FIGS. 5A and 5C, the shaft 50 constituting the axis 40 is rotatably supported within a bearing housing 53 via bearings 55A and 55B, and a stator 54 is provided in a cylindrical shape on an outer peripheral surface of the bearing housing 53. A rotor 51 is provided at a distal end portion 50a of the shaft 50, which protrudes through the bearing housing 53, and includes a disc-shaped lid portion 510 fixed to the shaft 50 and a cylindrical tube portion 511 extending from the outer peripheral portion of the lid portion 510 toward a base end side. A cylindrical magnet 52 is attached to an inner peripheral surface of the tube portion 511 of the rotor 51.

The lid portion 510 of the rotor 51 is fixed to the inner surface of the connecting plate 42 of the impeller 4 as described above. Furthermore, as shown in FIG. 4, the bearing housing 53 is fixed to a cover plate 72, which will be described later, through a support part 56 on the base end side. With such a structure, the shaft 50, the rotor 51, and the impeller 4 rotate with respect to the bearing housing 53 and stator 54, which are fixed to the cover plate 72 and do not rotate.

Here, it is preferable that the shaft 50 of the motor 5 and the rotor 51 to which the impeller 4 is attached be fixed by caulking, which has already been proposed by the present applicant in Japanese Unexamined Patent Application Publication No. 2007-283404. Specifically, as shown in FIG. 19A, the rotor 51 is attached to a lower support portion 94 that has an insertion hole through which the shaft 50 is inserted, and abuts to support a thick part 51a around an attachment hole 51b provided at a center of the lid 510 of the rotor 51 from below. Then, the shaft 50 is inserted and set into the attachment hole 51b of the rotor 51 and the insertion hole of the lower support portion 94 with a circumferential groove 50c facing upward, so that the thick portion 51a and the circumferential groove 50c are opposed to each other.

The circumferential groove 50c is formed near the end of the shaft 50, and is set by the lower support portion 94 at a position where the circumferential groove 50c and the thick portion 51a face each other. In this example, the thick portion 51a formed by burring is set so as to face upward, but it is also possible to set the thick portion 51a to face downward.

A pressure punch 92 is pressed down on the set shaft 50 and rotor 51 via a spacer jig 93 that contacts the thick part 51a from above, and the thick part 51a is compressed between the spacer jig 93 and the lower support portion 94. Thus, the thick part 51a is plastically deformed toward a center of the attachment hole and bites into a gap in the circumferential groove 50c of the opposing shaft 50. Finally, as shown in FIG. 19B, the plastically deformed thick part 51a fills the inside of the circumferential groove 50c, and the shaft 50 and rotor 51 are firmly assembled into a single body.

According to such caulking, the rotor 51 can be coaxially assembled to the shaft 50 with high accuracy. Even in the rotation at high speeds, there is no vibration and quietness can be maintained. Although the thick portion 51a is formed by burring so that an edge of the hole stands up in one direction, the thick portion 51a may also be formed into other shapes.

As shown in FIGS. 10A and 10B, the motor 5 is formed to be thinner, thereby making it possible to form the impeller 4 and the photocatalyst unit 1 to be thinner. According to the caulking method described above, the rotor 51 can be firmly and accurately fixed to a position nearest to a tip end 50a of the shaft 50. Furthermore, a distance between the bearings 55A and 55B is reduced, thereby achieving such reduction in thickness. The gap between the impeller 4 and the connecting plates 42 and 43 placed outside the impeller 4 can also be made small in this way. The above can be achieved by adopting a later-described fixing structure to the fixing structure of the blade plates 41 and the connecting plates 42 and 43, and the fixing structure of the rectifier plate 6 and the cover plates 71 and 72, in addition to the stable fixing of the motor 5 with the caulking structure described above.

The impeller 4 according to the present embodiment is composed of the impeller 4 serving as a blower fan. Here, the impeller serving as a cross flow fan may be used, and such an impeller may be rotated by a separate drive motor.

As shown in FIGS. 2 to 6, the photocatalyst filter 2 according to the present embodiment includes, in addition to the motor 5 and the impeller 4 that is the photocatalyst carrier, a rectifier plate 6 that extends in the circumferential direction on the outer peripheral side of the impeller 4 and forms a fluid passage 10 with the impeller 4, and a pair of upper and lower cover plates 71 and 72 that are respectively fixed to both ends of the rectifier plate 6 in the axial direction and are provided to sandwich the rectifier plate 6. An interrupted opening of the rectifier plate 6 functions as a discharge port 20 for discharging the fluid. As shown in FIG. 4, a supply hole 720 is provided in the center of one cover plate 72 to supply the fluid in the axial direction to the gap between each blade plate 41 of the impeller 4. Through the supply hole 720, the support part 56 of the motor 5 that rotates the impeller 4 extends to an outer surface of the cover plate 72 and is fixed to the outer surface.

For the fixing structure between the rectifier plate 6 and each of the cover plates 71 and 72, a strong structure is adopted which is similar to the fixing structure between each of the blade plates 41 constituting the impeller 4 and the connecting plates 42 and 43 described above. Specifically, a part of each end portion of the rectifier plate 6 (the upper end portion 6a and the lower end portion 6b in the vertical direction in the drawing) penetrates and protrudes through each of the through grooves 71c and 72c respectively formed in the cover plates 71 and 72. In addition, the protrusion (protrusion 61 on the upper end portion 6a, protrusion 62 on the lower end portion 6b) is bent in a direction intersecting the axial direction and parallel to a surface of the rectifier plate 6, and is fixed to the opening periphery of each of the through grooves 71c and 72c of the cover plates 71 and 72 in a crimped state.

Such fixing of the rectifier plate 6 and the cover plates 71 and 72 can be achieved by bending and deforming the protrusions 61 and 62 in a direction that intersects the axial direction and parallel to the surface of the rectifier plate 6 by caulking similar to the caulking performed on the impeller 4 described above (here, since the photocatalyst is not carried, the second caulking is not needed), so as to be fixed to the opening peripheries of the through grooves 71c and 72c of the cover plates 71 and 72. As a result, like the impeller 4, assembly of the rectifier plate 6 and the cover plates 71 and 72 has rigidity, can withstand wind pressure, maintains accuracy, and is made to be thin and compact (minimizes the gap with the impeller).

The light emitting unit 3 that emits light to the photocatalyst filter 2 is installed at a position outside the rectifier plate 6, as shown in FIGS. 2, 3, and 6, and is configured to emit the light inward to the axis 40 of the impeller 4 through a light projection through hole 60 formed in the rectifier plate 6. With this configuration, the light emitting unit 3 is prevented from interfering with the flow of air within the fluid passage 10. Accordingly, pressure loss can be reduced, deterioration due to dust or the like can be avoided, and assembly can be facilitated. In the present example, the light emitting element is placed inside the through hole 60, but is not limited thereto.

According to the arrangement of the light emitting unit 3, as shown in FIG. 6, the rectifier plate 6 is present in an area on a straight line L1 between a center point 120 of an opening surface of the fluid outlet 12 and a center point 600 of an inner opening surface of the light projection through hole 60 formed in the rectifier plate 6, and is configured to prevent light emitted inward from the light projection through hole 60 from being blocked by the rectifier plate 6 and from directly exiting from the fluid outlet 12 to the outside. It should be noted that the presence of the blower fan blade plates 41 (or cross flow fan blade plates) as in the present invention also exhibits the effect of causing the light from the light emitting unit 3 to hardly leak to the outside.

The light emitting unit 3 is configured to emit the light from the predetermined position toward the axis 40 of the impeller 4. Here, the light may be emitted in an oblique direction away from the axis 40, or may be emitted from diagonally above or below. However, it is preferable to emit the light in a direction perpendicular to the axial center of the axis 40 for applying the light uniformly to the entire surface of all the blade plates 41, that is, to both an upper surface of the blade plate 41 located on the upstream side in the fluid passage direction and a lower surface of the blade plate 41 located on the downstream side in the fluid passage direction.

The rectifier plate 6 also serves as a metal reflective member whose inner peripheral surface facing the impeller 4 serves as a reflective surface that reflects light. The reflective surface includes a metal material surface as it is or a surface processed into a mirror surface, a surface having an inner peripheral surface to which a mirror sheet is pasted, a surface coated with a reflective material, and the like.

As shown in FIGS. 1, 2, 3, and 5A to 5C, the housing 8 includes: divided cases 80 and 81 in a form of upper and lower halves that accommodate the photocatalyst filter 2 so as to wrap it up from above and below; a first cover plate 83 that is provided on an upper surface of the upper divided case 81 and accommodates a control board 19 that supplies power to the light emitting unit 3 and the motor 5 disposed on the upper surface, to control their operation; a second cover plate 82 that is provided on the entire lower surface of the divided case 80 with a gap therebetween so as to cover a fluid intake port 800 formed in a center of a bottom plate of the lower divided case 80. A lateral opening 82d in the gap between the cover plate 82 and the divided case 80 serves as the fluid inlet 11. This cover plate 82 is configured to prevent light from the light emitting unit 3 from leaking to the outside through the fluid intake port 800.

Notch grooves 80d and 81d are formed at positions corresponding to the discharge port 20 of the photocatalyst filter 2 at side boundary portions of the divided cases 80 and 81, respectively. A cylindrical outlet member 84 extending from the discharge port 20 and forming the fluid outlet 12 is attached to an inside of the opening formed by these notched grooves.

In addition to the present embodiment in which the first cover plate 83 for accommodating the control board 19 and the like is provided on the upper surface of the upper divided case 81, the divided cases 80 and 81 may be configured to be long laterally, and the control board and the like may be arranged side by side with the photocatalyst filter 2, as shown in FIG. 11. With this configuration, the first cover plate can be omitted, and the entire unit can be formed thinner.

It is also a preferable example that the outlet member 84 constituting the outlet 12 is provided with an attachment that can change a direction of the led-out fluid or connect a tube for conveying the fluid to another location. FIGS. 20A and 20B show an example in which an attachment 85A provided with a jet nozzle is removably attached to the outlet member 84 is provided. According to such an example, the attachment 85A allows the photocatalyst unit 1 placed in a breast pocket of clothing, for example, to eject cleaned fluid toward a human face upside.

FIG. 21 shows an example in which an attachment 85B that can bend a flow of fluid flowing out of the outlet member 84 by 90 degrees and discharge the fluid is also detachably provided. According to this example, cleaned fluid that flows out laterally from the photocatalyst unit 1 laid down on a desk or the like can be discharged upward to a human face.

FIG. 22 shows an example in which an attachment 85C to which a tube 86 can be attached is also detachably provided so that fluid exiting from the outlet member 84 can be supplied into the tube 86. According to this example, the cleaned fluid can be sent via the tube 86 to a tubular discharge portion 14 having a discharge hole 15 provided in a face shield 13 or a mask, for example.

Although the above embodiment has been described as an example in which the motor 5 is used to configure a fan that rotates the impeller 4, the present invention is not limited thereto. The motor 5 may be omitted and the blower fan-like impeller or the cross flow fan-like impeller that receives pressure from the fluid passing through the fluid passage 10 and rotates around a common axis like a windmill may be used.

For example, FIGS. 12 to 14 show examples in which a photocatalyst filter 2D is provided with a blower fan-like impeller 4D. Since these examples are basically the same as the photocatalyst filter 2 shown in FIGS. 1 to 9D described above, except that motor 5 is omitted, the same structures are provided with the same reference signs and the explanation thereof will be omitted.

Such an embodiment can function by being installed along a fluid passage 10 through which fluid is forcibly circulated by an electric fan 7 or the like in a ventilation duct or the like that ventilates a building. The embodiment can also function by additionally providing a fan for allowing fluid to flow inside the housing. The embodiment can also function by being installed in an environment with natural circulation. It is preferable that a housing different from the housing 8 shown in FIGS. 1 to 9D described above is appropriately adopted. Specifically, it is preferable to appropriately adopt a structure that easily guides fluid from the outside or a structure that additionally provides a fan allowing the fluid to flow inside.

FIGS. 15 to 18 show examples in which a photocatalyst filter 2E or a photocatalyst unit 1E is provided, which includes the cross flow fan-like impeller 4E. The motor that rotates the impeller 4E is omitted, and an electric fan 17 for allowing fluid to flow is provided at a position upstream of the fluid passage 10 (near the inlet 11 in this example) inside the housing 8E that also serves as a rectifier plate. The impeller 4E receives pressure from flow of the fluid, which is generated by the electric fan 17 and rotates like a windmill.

Two impellers 4E are provided in parallel, and the light emitting units 3 are respectively arranged at three locations along the rotation direction for each impeller 4E. Furthermore, since the blade plate 41E is long in the axial direction, a plurality of light emitting units 3 are provided at intervals along the axial direction.

Each blade plate 41E constituting the impeller 4E according to the present embodiment is also provided with connecting plates 42 and 43 at both ends of the plate 41E in the axial direction, similar to the respective above-described embodiments. As shown in FIG. 18, a fixing structure according to this example is also a strong fixing structure by caulking. The fixing structure is the same as that of each embodiment described above, and the explanation thereof will be omitted. The other configurations are basically the same as those in the photocatalyst filter 2 shown in FIGS. 1 to 9D described above, so that the same structures are provided with the same reference signs and the explanation thereof will be omitted.

Although each embodiment according to the present invention has been described above, the present invention is not limited to these embodiments, and can be implemented in various forms without departing from the scope of the claims. For example, directions in which the fluid flows may be set in the opposite directions for the respective embodiments.

### [Reference Signs List]

1, 1E photocatalyst unit
2, 2D, 2E photocatalyst filter
3 light emitting unit
4, 4D, 4E impeller
5 motor
6 rectifier plate
6a upper end portion
6b lower end portion
8, 8E housing
10 fluid passage
11 inlet
12 outlet
13 face shield
14 discharge portion
15 discharge hole
17 electric fan
19 control board
20 discharge port
21 photocatalyst
40 axis
41, 41E blade plate
41a, 41b end portion
42, 43 connecting plate
42c, 43c through groove
50 shaft
50a distal end portion
50c circumferential groove
51 rotor
51a thick part
51b attachment hole
53 bearing housing
54 stator
54 magnet
55A, 55B bearing
56 support part
60 through hole
61, 62 protrusion
71, 72 cover plate
71c, 72c through groove
80, 81 divided case
80d, 81d notch groove
82, 83 cover plate
82d opening
84 outlet member
85B attachment
85C attachment
86 tube
90, 91 pressure punch
92 pressure punch
93 spacer jig
94 support portion
120 center point
411, 412, 413, 414 protrusion
510 lid
511 tube portion
600 center point
720 supply hole
800 intake port

## Claims

1. A photocatalyst unit (1) comprising:
a photocatalyst filter (2) including a fluid passage (10) through which fluid passes, and a photocatalyst carrier, in the fluid passage, having a surface on which a photocatalyst is carried, the surface being in contact with the fluid; and
a light emitting unit (3) that is provided inside the photocatalyst filter and emits ultraviolet (UV) light or visible light to the surface of the photocatalyst carrier, the surface carrying the photocatalyst, wherein
the photocatalyst carrier includes a plurality of blade plates (41) that are made of metal, each of the plurality of blade plates having a surface on which the photocatalyst is carried, the plurality of blade plates constituting one of a blower fan like impeller (4) and a cross flow fan like impeller (4), which rotate about a common axis in response to pressure from the fluid passing through the fluid passage, or one of an impeller serving as a blower fan and an impeller serving as a cross flow fan, which rotate about a common axis by a motor drive,
the impeller includes a pair of connecting plates (42, 43) that connect to each of the blade plates at both ends thereof in the axial direction, each of the blade plates and the connecting plates are fixed in a manner that a part of each of the both ends of the blade plate penetrates and protrudes through a through groove (42c, 43c) formed in each of the connecting plates, and the part (411, 412, 413, 414) protruding is bent in a direction intersecting the axial direction and parallel to the surface of the blade plate so as to be crimped and fixed to an opening periphery of the through groove of the connecting plate, and
the light emitting unit is provided to emit the light to the blade plates of the impeller from a predetermined position in the impeller on an outer peripheral side from the blade plates, the blade plates being rotating.

2. The photocatalyst unit according to claim 1, wherein
the photocatalyst is also carried on a surface of each of the pair of connecting plates that constitute the impeller.

3. The photocatalyst unit according to claim 1 or 2, wherein
the photocatalyst filter includes a rectifier plate (6) that is made of metal and extends in a circumferential direction on the outer periphery side of the impeller to form the fluid passage with the impeller, and a pair of upper and lower cover plates (71, 72) respectively fixed to both ends of the rectifier plate in the axial direction to have the rectifier plate interposed therebetween, and
the rectifier plate and each of the cover plates are fixed in a manner that a part of each of the both ends of the rectifier plate penetrates and protrudes through a through groove (71c, 72c) formed in each of the cover plates, and the part protruding is bent in a direction intersecting the axial direction and parallel to a surface of the rectifier plate so as to be crimped and fixed to an opening periphery of the through groove of the cover plate.

4. The photocatalyst unit according to claim 3, wherein
the light emitting unit is provided at a position outside the rectifier plate and emits the light toward an axis of the impeller provided inside the rectifier plate through a projection through hole (60) that is provided in the rectifier plate for projecting the light.

5. The photocatalyst unit according to any one of claims 1 to 4, wherein
the rectifier plate is placed on a straight line (L1) between a center point (120) of an opening surface of a fluid outlet (12) provided in an outer circumferential direction of the blade plates and a center point (600) of an inner opening surface of the projection through hole (60) formed in the rectifier plate, so as to prevent the light emitted inward through the projection through hole from directly exiting from the fluid outlet.

6. A method for manufacturing a photocatalyst unit (1) that includes:
a photocatalyst filter (2) including a fluid passage (10) through which fluid passes, and a photocatalyst carrier, in the fluid passage, having a surface on which a photocatalyst is carried, the surface being in contact with the fluid, and
a light emitting unit (3) that is provided inside the photocatalyst filter and emits UV light or visible light to the surface of the photocatalyst carrier, the surface carrying the photocatalyst, the method comprising:
allowing the photocatalyst carrier to include a plurality of blade plates (40) that are made of metal, each of the plurality of blade plates having a surface on which the photocatalyst is carried, the plurality of the blade plates constituting one of a blower fan like impeller (4) and a cross flow fan like impeller (4), which rotate about a common axis in response to pressure from the fluid passing through the fluid passage, or one of an impeller serving as a blower fan and an impeller serving as a cross flow fan, which rotate about a common axis by a motor drive;
allowing the impeller to include a pair of connecting plates (42, 43) that connect to each of the blade plates at both ends in the axial direction;
fixing the blade plate and the connecting plates by allowing a part of each of the both ends of the blade plate to penetrate and protrude from a through groove (42c, 43c) formed in each of the connecting plates, and bending the part in a direction intersecting the axial direction and parallel to the surface of the blade plate to fix the part to an opening periphery of the through groove of the connecting plate by caulking; and
providing the light emitting unit to emit the light to the blade plates of the impeller from a predetermined position in the impeller on an outer peripheral side from the blade plates, the blade plates being rotating.

7. The method for manufacturing a photocatalyst unit according to claim 6, comprising:
roughening an entire surface of the impeller including each blade plate and the pair of connecting plates by roughening processing including etching processing; and
causing the photocatalyst to be carried on the surface roughened.

8. The method for manufacturing a photocatalyst unit according to claim 7, comprising:
performing another caulking on a portion fixed by the caulking previously performed after the roughing.

9. The method for manufacturing a photocatalyst unit according to any one of claims 6 to 8, comprising:
allowing the photocatalyst filter to include a rectifier plate (6) that is made of metal and extends in a circumferential direction on the outer peripheral side of the impeller to form the fluid passage with the impeller, and a pair of upper and lower cover plates (71, 72) respectively fixed to both ends of the rectifier plate in the axial direction to have the rectifier plate interposed therebetween; and
fixing the rectifier plate and the cover plates by allowing a part of each of the both ends of the rectifier plate to penetrate and protrude from a through groove (71c, 72c) formed in each of the cover plates, and bending and deforming the part in a direction intersecting the axial direction and parallel to the surface of the rectifier plate to fix the part to an opening periphery of the through groove of the cover plate by caulking.

## Patentansprüche

1. Photokatalysatoreinheit (1), die aufweist:
einen Photokatalysatorfilter (2), der einen Fluidkanal (10), durch den ein Fluid strömt, und einen Photokatalysatorträger in dem Fluidkanal aufweist, der eine Oberfläche aufweist, auf der ein Photokatalysator getragen wird, wobei die Oberfläche in Kontakt mit dem Fluid steht; und
eine lichtemittierende Einheit (3), die innerhalb des Photokatalysatorfilters vorgesehen ist und ultraviolettes (UV) Licht oder sichtbares Licht auf die Oberfläche des Photokatalysatorträgers emittiert, wobei die Oberfläche den Photokatalysator trägt, wobei der Photokatalysatorträger mehrere Flügelplatten (41) aufweist, die aus Metall bestehen, wobei jede der mehreren Flügelplatten eine Oberfläche aufweist, auf der der Photokatalysator getragen wird, wobei die mehreren Flügelplatten eines bilden von:
einem zu einem Gebläseventilator ähnlichen Laufrad (4) undr einem zu einem Querstromventilator ähnlichen Laufrad (4), die sich als Reaktion auf den Druck des durch den Fluidkanal strömenden Fluids um eine gemeinsame Achse drehen, oder einem als Gebläseventilator dienenden Laufrad und einem als Querstromventilator dienenden Laufrad, die sich durch einen Motorantrieb um eine gemeinsame Achse drehen,
das Laufrad ein Paar von Verbindungsplatten (42, 43) aufweist, die mit jeder der Flügelplatten an deren beiden Enden in der axialen Richtung verbunden sind, wobei jede der Flügelplatten und die Verbindungsplatten in einer Weise befestigt sind, dass ein Teil von jedem der beiden Enden der Flügelplatte durch eine Durchgangsnut (42c, 43c) dringt und vorsteht, die in jeder der Verbindungsplatten ausgebildet ist, und der vorstehende Teil (411, 412, 413, 414) in einer Richtung gebogen ist, die die axiale Richtung schneidet und parallel zur Oberfläche der Flügelplatte verläuft, so dass er an einem Öffnungsumfang der Durchgangsnut der Verbindungsplatte gekrimpt und befestigt ist, und
die lichtemittierende Einheit vorgesehen ist, um das Licht zu den Flügelplatten des Laufrads von einer vorbestimmten Position in dem Laufrad auf einer Außenumfangsseite der Flügelplatten zu emittieren, wobei die Flügelplatten rotieren.

2. Photokatalysatoreinheit nach Anspruch 1, wobei
der Photokatalysator auch auf einer Oberfläche jedes des Paars von Verbindungsplatten, die das Laufrad bilden, getragen wird.

3. Photokatalysatoreinheit nach Anspruch 1 oder 2, wobei
der Photokatalysatorfilter eine Gleichrichterplatte (6), die aus Metall besteht und sich in einer Umfangsrichtung auf der Außenumfangsseite des Laufrads erstreckt, um den Fluidkanal mit dem Laufrad zu bilden, und ein Paar oberer und unterer Abdeckplatten (71, 72) aufweist, die jeweils an beiden Enden der Gleichrichterplatte in der axialen Richtung befestigt sind, so dass die Gleichrichterplatte dazwischen angeordnet ist, und
die Gleichrichterplatte und jede der Abdeckplatten so befestigt sind, dass ein Teil jedes der beiden Enden der Gleichrichterplatte durch eine in jeder der Abdeckplatten ausgebildete Durchgangsnut (71c, 72c) dringt und vorsteht, und der vorstehende Teil in einer Richtung gebogen ist, die die axiale Richtung schneidet und parallel zu einer Oberfläche der Gleichrichterplatte verläuft, so dass er an einen Öffnungsumfang der Durchgangsnut der Abdeckplatte gekrimpt und befestigt ist.

4. Photokatalysatoreinheit nach Anspruch 3, wobei
die lichtemittierende Einheit an einer Position außerhalb der Gleichrichterplatte vorgesehen ist und das Licht zu einer Achse des innerhalb der Gleichrichterplatte vorgesehenen Laufrads durch ein Projektionsdurchgangsloch (60) emittiert, das in der Gleichrichterplatte zum Projizieren des Lichts vorgesehen ist.

5. Photokatalysatoreinheit nach einem der Ansprüche 1 bis 4, wobei
die Gleichrichterplatte auf einer geraden Linie (L1) zwischen einem Mittelpunkt (120) einer Öffnungsfläche eines Fluidauslasses (12), der in einer Außenumfangsrichtung der Flügelplatten vorgesehen ist, und einem Mittelpunkt (600) einer inneren Öffnungsfläche des in der Gleichrichterplatte ausgebildeten Projektionsdurchgangslochs (60) angeordnet ist, um zu verhindern, dass das durch das Projektionsdurchgangsloch nach innen emittierte Licht direkt aus dem Fluidauslass austritt.

6. Verfahren zur Herstellung einer Photokatalysatoreinheit (1), die aufweist:
einen Photokatalysatorfilter (2), der einen Fluidkanal (10), durch den ein Fluid strömt,
und einen Photokatalysatorträger in dem Fluidkanal aufweist, der eine Oberfläche aufweist, auf der ein Photokatalysator getragen wird, wobei die Oberfläche in Kontakt mit dem Fluid steht, und
eine lichtemittierende Einheit (3), die innerhalb des Photokatalysatorfilters vorgesehen ist und UV-Licht oder sichtbares Licht auf die Oberfläche des Photokatalysatorträgers emittiert, wobei die Oberfläche den Photokatalysator trägt, wobei das Verfahren aufweist:
Ermöglichen, dass der Photokatalysatorträger mehrere Flügelplatten (40) aufweist, die aus Metall hergestellt sind, wobei jede der mehreren Flügelplatten eine Oberfläche aufweist, auf der der Photokatalysator getragen wird, wobei die mehreren Flügelplatten eines bilden von: einem zu einem Gebläseventilator ähnlichen Laufrad (4) und einem zu einem Querstromventilator ähnlichen Laufrad, die sich als Reaktion auf den Druck des durch den Fluidkanal strömenden Fluids um eine gemeinsame Achse drehen, oder einem als Gebläseventilator dienenden Laufrad und einem als Querstromventilator dienenden Laufrad, die sich durch einen Motorantrieb um eine gemeinsame Achse drehen;
Ermöglichen, dass das Laufrad ein Paar von Verbindungsplatten (42, 43) aufweist, die mit jeder der Flügelplatten an beiden Enden in der axialen Richtung verbunden sind;
Befestigen der Flügelplatte und der Verbindungsplatten durch Ermöglichen, dass ein Teil jedes der beiden Enden der Flügelplatte durch eine Durchgangsnut (42c, 43c), die in jeder der Verbindungsplatten ausgebildet ist, dringt und vorsteht, und Biegen des Teils in einer Richtung, die die axiale Richtung schneidet und parallel zur Oberfläche der Flügelplatte verläuft, um den Teil an einem Öffnungsumfang der Durchgangsnut der Verbindungsplatte durch Verstemmen zu befestigen; und
Bereitstellen der lichtemittierenden Einheit, um das Licht zu den Flügelplatten des Laufrads von einer vorbestimmten Position in dem Laufrad auf einer Außenumfangsseite von den Flügelplatten zu emittieren, wobei die Flügelplatten rotieren.

7. Verfahren zur Herstellung einer Photokatalysatoreinheit nach Anspruch 6, das aufweist:
Aufrauen der gesamten Oberfläche des Laufrads, einschließlich jeder Flügelplatte und des Paars von Verbindungsplatten, durch eine Aufrauungsbearbeitung, die eine Ätzbearbeitung umfasst; und
Bewirken, dass der Photokatalysator auf der aufgerauten Oberfläche getragen wird.

8. Verfahren zur Herstellung einer Photokatalysatoreinheit nach Anspruch 7, das aufweist: Durchführen eines weiteren Verstemmens an einem Abschnitt, der durch das zuvor nach dem Aufrauen durchgeführte Verstemmen fixiert wurde.

9. Verfahren zur Herstellung einer Photokatalysatoreinheit nach einem der Ansprüche 6 bis 8, das aufweist:
Ermöglichen, dass der Photokatalysatorfilter eine Gleichrichterplatte (6), die aus Metall besteht und sich in einer Umfangsrichtung auf der Außenumfangsseite des Laufrads erstreckt, um den Fluidkanal mit dem Laufrad zu bilden, und ein Paar von oberen und unteren Abdeckplatten (71, 72) aufweist, die jeweils an beiden Enden der Gleichrichterplatte in der axialen Richtung befestigt sind, so dass die Gleichrichterplatte dazwischen angeordnet ist; und
Befestigen der Gleichrichterplatte und der Abdeckplatten durch Ermöglichen, dass ein Teil von jedem der beiden Enden der Gleichrichterplatte durch eine Durchgangsnut (71c, 72c), die in jeder der Abdeckplatten ausgebildet ist, dringt und vorsteht, und Biegen und Verformen des Teils in einer Richtung, die die axiale Richtung schneidet und parallel zur Oberfläche der Gleichrichterplatte ist, um den Teil an einem Öffnungsumfang der Durchgangsnut der Abdeckplatte durch Verstemmen zu befestigen.

## Revendications

1. Unité de photocatalyseur (1), comprenant :
un filtre photocatalytique (2) présentant un passage de fluide (10) où circule un fluide, et un support de photocatalyseur, dans le passage de fluide, ayant une surface sur laquelle un photocatalyseur est supporté, ladite surface étant en contact avec le fluide ; et
une unité électroluminescente (3) disposée à l'intérieur du filtre photocatalytique et émettant une lumière ultraviolette (UV) ou une lumière visible vers la surface du support de photocatalyseur, la surface supportant le photocatalyseur, où
le support de photocatalyseur comprend une pluralité de plaquettes de pale (41) en métal, chacune des plaquettes de pale ayant une surface sur laquelle le photocatalyseur est supporté, la pluralité de plaquettes de pale constituant un rotor (4) semblable à un ventilateur soufflant ou bien un rotor (4) semblable à un ventilateur à flux transversal, tournant autour d'un axe commun en réaction à la pression du fluide circulant dans le passage de fluide, ou encore un rotor servant de ventilateur soufflant ou bien un rotor servant de ventilateur à flux transversal, tournant autour d'un axe commun par entraînement de moteur,
le rotor comprend une paire de plaques de raccord (42, 43) raccordées à chacune des plaquettes de pale aux deux extrémités de celles-ci dans la direction axiale, chacune des plaquettes de pale et les plaques de raccord étant fixées de sorte qu'une partie de chacune des deux extrémités de la plaquette de pale pénètre dans, et fait saillie d'une rainure traversante (42c, 43c) formée dans chacune des plaques de raccord, et que la partie (411, 412, 413, 414) en saillie est incurvée dans une direction croisant la direction axiale et parallèle à la surface de la plaquette de pale, de manière à être sertie et fixée à une périphérie d'ouverture de la rainure traversante de la plaque de raccord, et
l'unité électroluminescente est prévue pour émettre de la lumière vers les plaquettes de pale du rotor depuis un emplacement prédéterminé dans le rotor sur un côté périphérique extérieur des plaquettes de pale, lesdites plaquettes de pale étant rotatives.

2. Unité de photocatalyseur selon la revendication 1, où
le photocatalyseur est également supporté sur une surface de chaque plaque de la paire de plaques de raccord constituant le rotor.

3. Unité de photocatalyseur selon la revendication 1 ou la revendication 2, où
le filtre photocatalytique comprend une plaque de redressement (6) en métal s'étendant dans la direction circonférentielle sur le côté de périphérie extérieure du rotor pour former le passage de fluide avec le rotor, et une paire de plaques de couverture, supérieure et inférieure (71, 72), fixées chacune à l'extrémité respective des deux extrémités de la plaque de redressement dans la direction axiale, de sorte que la plaque de redressement leur est intercalée, et
la plaque de redressement et chacune des plaques de couverture sont fixées de sorte qu'une partie de chacune des deux extrémités de la plaque de redressement pénètre dans, et fait saillie d'une rainure traversante (71c, 72c) formée dans chacune des plaques de couverture, et la partie en saillie est incurvée dans une direction croisant la direction axiale et parallèle à une surface de la plaque de redressement, de manière à être sertie et fixée à une périphérie d'ouverture de la rainure traversante de la plaque de couverture.

4. Unité de photocatalyseur selon la revendication 3, où
l'unité électroluminescente est disposée à un emplacement de l'extérieur de la plaque de redressement et émet la lumière en direction de l'axe du rotor placé à l'intérieur de la plaque de redressement, par un orifice de projection (60) prévu dans la plaque de redressement pour projeter la lumière.

5. Unité de photocatalyseur selon l'une des revendications 1 à 4, où
la plaque de redressement est placée sur une ligne droite (L1) entre un point central (120) d'une surface d'ouverture d'une sortie de fluide (12) prévue dans la direction circonférentielle extérieure des plaquettes de pale et un point central (600) d'une surface d'ouverture intérieure de l'orifice de projection (60) formé dans la plaque de redressement, de manière à empêcher la lumière émise vers l'intérieur par l'orifice de projection de sortir directement de la sortie de fluide.

6. Procédé de fabrication d'une unité de photocatalyseur (1) comprenant :
un filtre photocatalytique (2) présentant un passage de fluide (10) où circule un fluide, et un support de photocatalyseur, dans le passage de fluide, ayant une surface sur laquelle un photocatalyseur est supporté, ladite surface étant en contact avec le fluide ; et
une unité électroluminescente (3) disposée à l'intérieur du filtre photocatalytique et émettant une lumière UV ou une lumière visible vers la surface du support de photocatalyseur, la surface supportant le photocatalyseur, ledit procédé comprenant :
la réalisation des conditions d'inclusion, par le support de photocatalyseur, d'une pluralité de plaquettes de pale (40) en métal, chacune des plaquettes de pale ayant une surface sur laquelle le photocatalyseur est supporté, la pluralité de plaquettes de pale constituant un rotor (4) semblable à un ventilateur soufflant ou bien un rotor (4) semblable à un ventilateur à flux transversal, tournant autour d'un axe commun en réaction à la pression du fluide circulant dans le passage de fluide, ou encore un rotor servant de ventilateur soufflant ou bien un rotor servant de ventilateur à flux transversal, tournant autour d'un axe commun par entraînement de moteur ;
la réalisation des conditions d'inclusion, par le rotor, d'une une paire de plaques de raccord (42, 43) raccordées à chacune des plaquettes de pale aux deux extrémités de celles-ci dans la direction axiale ;
la fixation de la plaquette de pale et des plaques de raccord en permettant à une partie de chacune des deux extrémités de la plaquette de pale de pénétrer dans, et de faire saillie d'une rainure traversante (42c, 43c) formée dans chacune des plaques de raccord, et l'incurvation de ladite partie dans une direction croisant la direction axiale et parallèle à la surface de la plaquette de pale, pour fixer par matage ladite partie à une périphérie d'ouverture de la rainure traversante de la plaque de raccord ; et
la préparation de l'unité électroluminescente pour émettre de la lumière vers les plaquettes de pale du rotor depuis un emplacement prédéterminé dans le rotor sur un côté périphérique extérieur des plaquettes de pale, lesdites plaquettes de pale étant rotatives.

7. Procédé de fabrication d'une unité de photocatalyseur selon la revendication 6, comprenant :
la rugosification de la surface entière du rotor, comprenant chaque plaquette de pale et la paire de plaques de raccord, par un processus de rugosification comprenant un processus de gravure ; et
la réalisation des conditions de support du photocatalyseur sur la surface rugosifiée.

8. Procédé de fabrication d'une unité de photocatalyseur selon la revendication 7, comprenant :
l'exécution d'un nouveau matage sur une partie fixée par le matage réalisé antérieurement, après rugosification.

9. Procédé de fabrication d'une unité de photocatalyseur selon l'une des revendications 6 à 8, comprenant :
la réalisation des conditions d'inclusion par le filtre photocatalytique d'une plaque de redressement (6) en métal s'étendant dans la direction circonférentielle sur le côté de périphérie extérieure du rotor pour former le passage de fluide avec le rotor, et d'une paire de plaques de couverture, supérieure et inférieure (71, 72), fixées chacune à l'extrémité respective des deux extrémités de la plaque de redressement dans la direction axiale, de sorte que la plaque de redressement leur est intercalée ; et
la fixation de la plaque de redressement et de chacune des plaques de couverture de sorte qu'une partie de chacune des deux extrémités de la plaque de redressement pénètre dans, et fait saillie d'une rainure traversante (71c, 72c) formée dans chacune des plaques de couverture, et l'incurvation et la déformation de la partie dans une direction croisant la direction axiale et parallèle à la surface de la plaque de redressement, pour fixer par matage ladite partie à une périphérie d'ouverture de la rainure traversante de la plaque de couverture.
